# EUROPÄISCHE PATENTSCHRIFT

(11) **EP 0 475 137 B1**
(45) Veröffentlichungstag und Bekanntmachung des Hinweises auf die Patenterteilung: **28.12.1994**
(21) Anmeldenummer: 91113983.0
(22) Anmeldetag: 21.08.1991
(51) Int. Cl.: C07C 51/60, C07C 53/42, C07C 57/66

(54) **Verfahren zur Herstellung von Carbonsäurechloriden**
Process for the preparation of carboxylic acid chlorides
Procédé pour la préparation de chlorures d'acides

(30) Priorität: 11.09.1990 DE 4028774
(43) Veröffentlichungstag der Anmeldung: 18.03.1992
(73) Patentinhaber: BASF Aktiengesellschaft, 67063 Ludwigshafen (DE)
(72) Erfinder: Ksoll, Peter, Dr., W-6915 Dossenheim (DE); Reuther, Wolfgang, Dr., W-6900 Heidelberg (DE); Hohmann, Andreas, Dr., W-6700 Ludwigshafen (DE); Wittmer, Peter, W-6730 Neustadt (DE)

(56) Entgegenhaltungen:
- EP-A- 0 367 050

## Beschreibung

Die vorliegende Erfindung betrifft ein neues und verbessertes Verfahren zur Herstellung von Carbonsäurechloriden aus Carbonsäuren und Phosgen an einer stehenden Katalysatorphase aus einem Katalysator-Addukt aus Phosgen und N,N-disubstituierten Formamid, wobei das Formamid zu 0,1 bis 95 mol-% mit Phosgen beladen ist.

Carbonsäurechloride lassen sich gut durch Umsetzung der entsprechenden Carbonsäuren mit Phosgen herstellen. Die Reaktion bedarf der Katalyse. Als Katalysatoren kommen z.B. Carboxamide, vorzugsweise N-Alkylformamide zum Einsatz (DE-A-34 39 937).

Die Spanne der Alkylgröße reicht bei den N,N-Dialkylformamiden von Dimethylformamid bis zu Formamiden mit 30 C-Atomen (EP-A-0 050 779, DE-A-29 50 155, DE-A-19 31 074).

Durch die Wahl des Katalysatorsystems wird der Ablauf der Phosgenierung einer Carbonsäure zum Carbonsäurechlorid sowie die Aufarbeitung des Ansatzes entscheidend beeinflußt.

Alternativ zur Filtration von teerhaltigen Rohprodukten wäre in einigen Fällen auch eine destillative Aufarbeitung des katalysatorhaltigen Produktes denkbar. Die Destillation der entstandenen Säurechloride ist aber nicht nur ein energie- und zeitaufwendiger Vorgang, sondern birgt auch noch eine Reihe weiterer Nachteile.

Viele längerkettige Säurechloride lassen sich nicht ohne teilweise Zersetzung destillieren. Weiter ist bekannt, daß durch Zersetzung des im Destillationssumpf befindlichen Katalysators die destillierten Produkte verunreinigt werden können. Größere Mengen an Katalysatorrückstand stellen bei der Destillation ein Sicherheitsrisiko dar, weil in der Hitze die Gefahr einer spontanen Zersetzung besteht.

Bei der Produktaufarbeitung verunreinigter Ansätze vermindert sich sowohl durch Filtration als auch durch Destillation die Aktivität des Katalysators stark. In den meisten Fällen wird der eingesetzte Katalysator unbrauchbar, er kann also nicht wiederverwendet werden.

Sowohl die Destillation als auch die Filtration von katalysatorhaltigen Carbonsäurechloriden stellen also unvorteilhafte Aufarbeitungsmethoden dar. Wegen des durch die Aufarbeitung bedingten Katalysatorverlustes muß dessen Aufwandmenge so gering wie möglich ausfallen.

In der DE-A-29 50 155 wird als Katalysator Diisobutylformamid verwendet, das in jeder Phase der Umsetzung im Reaktionsansatz löslich ist. Soll auf eine abschließende Destillation des Säurechlorids verzichtet werden, muß aus Gründen der Produktreinheit der Anteil des löslichen Katalysators auf ein Minimum beschränkt werden. Auch bei diesem Katalysatorsystem ist eine Wiederverwendung des Katalysators ausgeschlossen, da er mit dem Produkt ausgetragen wird.

Es ist auch bekannt, daß die Umsetzungen mit Phosgen um so effektiver ablaufen, je größer der Katalysatoranteil ist. Umgekehrt bedingen geringe Katalysatormengen entweder eine schlechte Ausnutzung des eingegasten Phosgens oder lange Eingaszeiten.

In der DE-A-22 40 883 wird die Herstellung von Carbonsäurechloriden mit äquimolaren Mengen Carbonsäure und Katalysator beschrieben. Zur Abtrennung und Wiedergewinnung der großen Katalysatormenge ist allerdings die abschließende Zugabe des 3- bis 4-fachen Reaktionsvolumens an Benzol mit anschließender Destillation der benzolischen Produktlösung notwendig.

Den Einsatz großer Mengen Katalysator beschreibt auch JP-10 613/68 bei der Herstellung von Linolsäurechlorid mit 10 bis 50 Mol.% Dimethylformamid, aber auch 1 bis 10 Äquivalenten Dimethylformamid, bezogen auf eingesetzte Linolsäure. Das entstandene Säurechlorid muß destilliert und zum Teil durch eine Behandlung mit Aktivkohle zusätzlich gereinigt werden. Die erneute Verwendung der großen Katalysatormengen ist nicht vorgesehen.

Bei der Umsetzung von Carbonsäuren mit Phosgen zu den entsprechenden Säurechloriden stellt sich bekanntermaßen das Problem, überschüssiges Phosgen aus dem rohen Säurechlorid zu entfernen.

Nach dem Stand der Technik kann phosgenhaltiges Carbonsäurechlorid durch mehrstündiges Strippen mit Stickstoff und/oder durch Anlegen eines leichten Vakuums von Phosgen befreit werden. Dieser Vorgang ist zeitraubend und verschlechtert die Raum-Zeit-Ausbeute des Verfahrens erheblich.

In der DE-A-29 50 155 wird das überschüssige Phosgen mit dem ersten Teil des destillierten Säurechlorids überdestilliert. Neben einer auch hier zu beobachtenden Verschlechterung der Raum-Zeit-Ausbeute erfordert diese Vorgehensweise zusätzlichen apparativen und analytischen Aufwand.

Aus der DE-A-22 40 883 ist eine Aufarbeitung bekannt, bei der vor der Destillation die verdünnte Reaktionslösung kurz mit Eiswasser gewaschen wird. Angesichts der Hydrolyseempfindlichkeit von Carbonsäurechloriden ist dieses Verfahren für den technischen Maßstab problematisch.

Auch bei dem aus JP 10613/68 bekannten Verfahren muß überschüssiges Phosgen durch eine destillative Aufarbeitung des rohen Säurechlorids entfernt werden.

Aus der DE-A-38 36 967 ist ein Verfahren zur Herstellung von höheren Carbonsäurechloriden bekannt, indem das N,N-Dialkylformamid zu 100 % mit Phosgen beladen wurde. Bei dieser Verfahrensweise entsteht ein nicht unerheblicher Phosgenverlust.

Aus der DE-A-40 12 781 ist ein Verfahren zur Herstellung von Carbonsäurechloriden bekannt, nachdem man Carbonsäurechloride sowohl diskontinuierlich als auch kontinuierlich herstellen kann. Hierbei wird der Katalysator während der gesamten Reaktionsdauer in der Säure bzw. dem Säurechlorid durch Rühren dispergiert. Durch die sich anschließende Phasentrennung verschlechtert sich die Raum-Zeit-Ausbeute des Verfahrens. Die lange Kontaktzeit der Produkte I mit dem Katalysator-System bei erhöhter Temperatur bewirken Qualtitätseinbußen des Produkts z.B. bezüglich der Farbzahl. Zudem ist eine Umsetzung bei Temperaturen unterhalb des Schmelzpunktes längerkettigter Carbonsäuren wie z.B. Stearinsäure ohne Lösungsmittel nicht möglich.

Die disperse Verteilung des Katalysators in der Säure bzw. in Säurechlorid hat zur Folge, daß der Abgasstrom CO₂/HCl nicht unwesentliche Mengen an Phosgen > 1,0 % enthält.

Der Erfindung lag nun die Aufgabe zugrunde, ein Verfahren zur Herstellung von Carbonsäurechloriden zu finden, das den vorgenannten Nachteilen abhilft.

Demgemäß wurde ein neues und verbessertes Verfahren zur Herstellung von Carbonsäurechloriden der allgemeinen Formel I 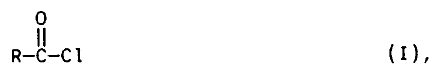
in der R für C₁- bis C₃₀-Alkyl, C₂- bis C₃₀-Alkenyl und C₂- bis C₃₀-Alkinyl steht, aus im wesentlichen äquimolaren Mengen einer Carbonsäure der allgemeinen Formel II 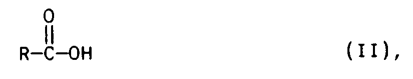
in der R die oben genannten Bedeutungen hat, und Phosgen COCl₂ (III), in Gegenwart eines Katalysator-Addukts aus Phosgen und N,N-disubstituiertem Formamid der allgemeinen Formel IV 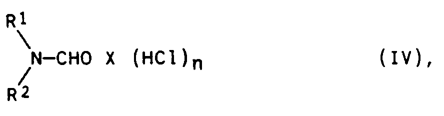
in der R¹ und R² unabhängig voneinander C₁- bis C₃-Alkyl oder R¹ und R² gemeinsam eine C₄- oder C₅-Alkylenkette bedeuten, die gegebenenfalls durch ein Sauerstoffatom oder ein Stickstoffatom, das eine C₁- bis C₃-Alkyl-gruppe oder CHO trägt, unterbrochen sein kann und n 0, 1 oder 2 bedeutet, gefunden, welches dadurch gekennzeichnet ist, daß man die Reaktion mit einer stehenden Phase des Katalysator-Adduktes durchführt und das Formamid (IV) zu 0,1 bis 95 mol-% mit Phosgen belädt.

Die Carbonsäurechloride I sind nach folgender Methode erhältlich:
Man kann die Komponenten, die Carbonsäure II und das Phosgen III, stufenweise oder kontinuierlich bei Temperaturen von 20 bis 140°C, vorzugsweise 45 bis 100°C , besonders bevorzugt bei 30 bis 80°C und Drucken von 0,01 bis 50 bar, vorzugsweise von 0,1 bis 5 bar, besonders bevorzugt bei Normaldruck (Atmosphärendruck) ca. 1 bar in einem - mit einer stehenden Phase eines Katalysator Adduktes aus Phosgen und eines N,N-disubstituierten Formamids IV gefüllten - Reaktor leiten und zur Reaktion bringen.

Bei der stufenweisen Reaktion kann man das Katalysator-Addukt durch Einleiten von Phosgen III damit beladen und anschließend eine Carbonsäure II durchleiten, bis das Katalysator-Addukt entladen ist und die Prozedur wiederholen etc.

Bei der kontinuierlichen Variante kann man die Carbonsäure II und Phosgen III in im wesentlichen äquimolaren Mengen, also im Molverhältnis von 0,8 : 1 bis 1,2:1, bevorzugt von 0,95 : 1 bis 1,05 : 1, besonders bevorzugt von 1 : 1 in die stehende Phase des Katalysator-Adduktes einleiten.

Bei der Durchführung sollte die Strömungsgeschwindigkeit der Edukte II und III so gewählt werden, daß in der stehenden Katalysator-Addukt-Phase nur geringe oder keine Turbulenzen auftreten. Besonders bevorzugt sind laminare Strömungsverhältnisse in der stehenden Katalysator-Phase.

Nach dem Durchgang der Edukte II und III durch die stehende Katalysatorphase trennt sich in aller Regel das Carbonsäurechlorid I als Produkt oberhalb der stehenden Katalysatorphase automatisch ab und kann in einem Vorratsgefäß aufgefangen werden und gegebenenfalls durch übliche Reinigungsmethoden wie Destillation oder Kristallisation nachgereinigt werden. Im allgemeinen ist die Produktqualität jedoch so, daß sich eine Reinigung erübrigt.

Als Reaktoren eignen sich alle üblichen Reaktoren wie Rohrreaktoren, Schlangenreaktoren, Kessel, mit der Maßgabe, daß eine Katalysatorschicht von einer Höhe bereitgestellt wird, die bei laminarer Durchströmung von Phosgen keine Dispersion mit der Säure bzw. dem Säurechlorid ausbildet, bevorzugt stehende Rohrreaktoren, denen von unten die Edukte zugeführt werden.

Der Reaktionsverlauf kann mit Hilfe von physikalischen Methoden kontrolliert werden, wie Dichte-, Leitfähigkeits- und Chlorid-Gehalts-Messungen.

Als Leitfähigkeitsmeßsonden eignen sich induktiv oder direkt messende Elektroden, bevorzugt induktiv messende.

Die Leitfähigkeitsmeßsonden werden im Reaktor positioniert, bevorzugt in der Katalysatorphase.

Die Leitfähigkeitswerte während der Reaktion lagen zwischen 10 und 15 mS/cm (Millisiemens/Zentimeter, Dimension x = mS/cm), bevorzugt zwischen 10,5 und 14,5 mS/cm, besonders bevorzugt zwischen 11 und 14 mS/cm.

Das Verhältnis der Höhe zum Durchmesser des Reaktors und/oder des stehenden Katalysator-Adduktes beträgt größer oder gleich 0,2, also 0,2 bis 500, bevorzugt größer oder gleich 1, also 1 bis 200, besonders bevorzugt 10 bis 50.

Die stehende Katalysatorphase, also das Katalystor-Addukt aus Phosgen und N,N-substituiertem Formamid x(HCl)ₙ) IV mit n = (0, 1, 2) kann aus einem Gemisch folgender Verbindungen bestehen: 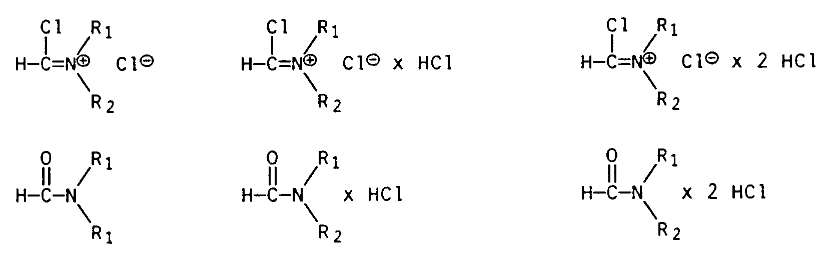
Der Beladungsgrad des Katalysators liegt zwischen 0,1 und 95 mol-%, vorzugsweise 10 bis 80 mol-%, besonders 20 bis 70 mol-%.

Der Beladungsgrad des Katalysators kann kontinuierlich durch Messung der Dichte, der Viskosität, der Leitfähigkeit oder des Chlorid-Gehalts der im Trenngefäß anfallenden schwereren Phase bestimmt werden.

Die Phasentrennung erfolgt im Reaktor bei Temperaturen von 20 bis 100°C, vorzugsweise 30 bis 80°C, besonders 40 bis 70°C.

Die eingesetzte Phosgenmenge (III) ist im wesentlichen äquimolar zur Carbonsäure (II).

Da das Reaktionsabgas neben Kohlendioxid und Chlorwasserstoff nur unwesentliche Mengen Phosgen enthält (0,0001 bis 0,1 %) ist eine Kondensation des Phosgens nicht erforderlich. Die Abgase können direkt der Abgaswäsche zugeführt werden. Zur Kühlung des Abgases sind deshalb nur solche Temperaturen notwendig, die das gebildete Wertprodukt möglichst vollständig kondensieren.

Dem Reaktionsgemisch kann, zur besseren Phasentrennung oder zur Umsetzung fester Säuren, ein unter diesen Reaktionsbedingungen inertes Lösungsmittel zugesetzt werden, wie z.B. gesättigte aliphatische Kohlenwasserstoffe, Ether, Acetonitril, Benzol, Toluol oder Cyclohexan.

Ein besonderer Vorteil des Verfahrens besteht darin, daß auch feste Säuren bei Temperaturen unterhalb ihrer Schmelztemperaturen ohne Lösungsmittel umgesetzt werden können.

Das Carbonsäurechlorid fällt in hoher Ausbeute und in hoher Reinheit an. Es kann häufig ohne weitere Reinigung weiterverwendet werden. In einigen Fällen ist zur Erlangung hochreiner Produkte eine einfache Destillation erforderlich. Die Carbonsäurechloride fallen nach der Umsetzung phosgenfrei an und alle Maßnahmen zur Entphosgenierung des rohen Säurechlorids entfallen.

Das erfindungsgemäße Verfahren zur Herstellung von Säurechloriden aus aliphatischen Carbonsäuren eignet sich vor allem für Monocarbonsäuren, d.h. zur Herstellung von Verbindungen mit der allgemeinen Formel RCOX, wobei R eine aliphatische Kohlenwasserstoffgruppe darstellt und X für Chlor steht. Die aliphatische Gruppe kann geradkettig oder verzweigt, gesättigt, olefinisch oder acetylenisch ungesättigt sein. Besonders bevorzugt sind aliphatische Carbonsäuren mit 1 bis 30, insbesondere 1 bis 20 Kohlenstoffatomen.

Als N,N-Dialkylformamide eignen sich Dimethyl-, Ethyl-methyl-, Methyl-n-propyl-, Methyl-iso-propyl-, Diethyl-, Ethyl-n-propyl, Ethyl-isopropyl-, Di-n-propyl-, n-Propyl-iso-propyl- und Di-iso-propyl-formamid, bevorzugt sind Dimethyl- und Diethylformamid, besonders bevorzugt ist Diethylformamid.

### Beispiele

### Beladung des Katalysators

In einem mit Raschigringen gefüllten 2-l-Glasrohrreaktor werden 7 Mol (707 g) Diethylformamid vorgelegt. Es wird bei 60°C solange durch Zugasen von Phosgen das Katalysator-Addukt gebildet, bis die Leitfähigkeit einen Wert von 14 mS/cm erreicht, was einem Beladungszustand von 95 % entspricht.

### Beispiel 1

### Herstellung von Stearinsäurechlorid

Geschmolzene Stearinsäure wurde mit einer Rate von 2,3 Mol/h in den vorbereiteten Katalysator, der eine Temperatur von 60°C hatte, zudosiert. Gleichzeitig wurde Phosgen so zugegast, daß der Leitfähigkeitswert zwischen 11 bis 14 mS/cm lag, was einem Beladungszustand von 40 bis 95 % entsprach.

Das Stearinsäurechlorid verließ nach ca. 20 min den Reaktor und wurde auf 25°C gekühlt und wie üblich durch Phasentrennung gewonnen. Man erhält 99,7 % Stearinsäurechlorid, IFZ: 5.

### Beispiel 2

### Herstellung von Ölsäurechlorid

Ölsäure wurde mit einer Rate von 1,3 Mol/h in den vorbereiteten Katalysator, der eine Temperatur von 60°C hatte, zudosiert. Gleichzeitig wurde Phosgen so zugegast, daß der Leitfähigkeitswert zwischen 11 und 14 mS/cm lag, was einem Beladungszustand von 40 bis 95 % entsprach.

Das Ölsäurechlorid verließ nach ca. 20 min den Reaktor und wurde auf 25°C gekühlt und wie üblich durch Phasentrennung gewonnen. Man erhielt 96,0 % Ölsäurechlorid, IFZ: 30.

### Beispiel 3

### Herstellung von 2-Ethylhexansäurechlorid

2-Ethylhexansäure wurde mit einer Rate von 2,5 Mol/h in den vorbereiteten Katalysator, der eine Temperatur von 60°C hatte, zudosiert. Gleichzeitig wurde Phosgen so zugegast, daß der Leitfähigkeitswert zwischen 11 und 14 mS/cm lag, was einem Beladungszustand von 40 bis 95 % entsprach.

Das 2-Ethylhexansäurechlorid verließ nach ca. 20 min den Reaktor und wurde auf 25°C gekühlt und wie üblich durch Phasentrennung gewonnen. Man erhielt 97,5 % 2-Ethylhexansäurechlorid, IFZ: 26.

## Patentansprüche

1. Verfahren zur Herstellung von Carbonsäurechloriden der allgemeinen Formel I 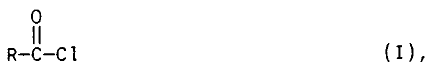 in der R für C₁- bis C₃₀-Alkyl, C₂- bis C₃₀-Alkenyl und C₂- bis C₃₀-Alkinyl steht, aus im wesentlichen äquimolaren Mengen einer Carbonsäure der allgemeinen Formel II 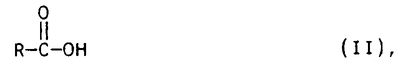 in der R die oben genannten Bedeutungen hat, und Phosgen COCl₂ (III), in Gegenwart eines Katalysator-Addukts aus Phosgen und N,N-disubstituiertem Formamid oder deren Hydrochloride der allgemeinen Formel IV 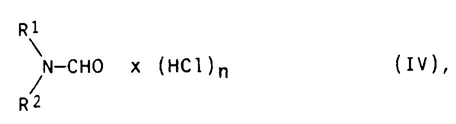 in der R¹ und R² unabhängig voneinander C₁- bis C₃-Alkyl oder R¹ und R² gemeinsam eine C₄- oder C₅-Alkylenkette bedeuten, die gegebenenfalls durch ein Sauerstoffatom oder ein Stickstoffacom, das eine C₁- bis C₃-Alkylgruppe oder CHO trägt, unterbrochen sein kann und n 0, 1 oder 2 bedeutet, dadurch gekennzeichnet, daß man die Reaktion mit einer stehenden Phase des Katalysator-Adduktes durchführt und das Formamid (IV) zu 0,1 bis 95 mol-% mit Phosgen belädt.

2. Verfahren nach Anspruch 1, dadurch gekennzeichnet, daß man die Umsetzung stufenweise oder kontinuierlich durchführt und das Formamid (IV) zu 0,1 bis 95 mol-% mit Phosgen belädt.

3. Verfahren nach Anspruch 1, dadurch gekennzeichnet, daß man die Umsetzung stufenweise oder kontinuierlich durchführt.

4. Verfahren nach Anspruch 1, dadurch gekennzeichnet, daß man als N,N-Dialkylformamid N,N-Dimethylformamid, Methyl-ethylformamid oder Diethylformamid verwendet.

5. Verfahren nach Anspruch 1, dadurch gekennzeichnet, daß man als N,N-Dialkylformamid N,N-Diethylformamid verwendet.

## Claims

1. A process for preparing a carbonyl chloride of the formula I 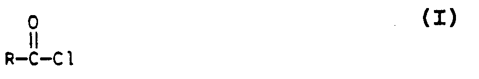 where R is C₁-C₃₀-alkyl, C₂-C₃₀-alkenyl or C₂-C₃₀-alkynyl, from essentially equimolar amounts of a carboxylic acid of the formula II 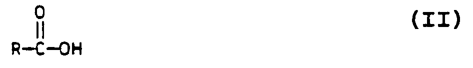 where R has the abovementioned meanings, and phosgene COCl₂ (III), in the presence of a catalytic adduct of phosgene and N,N-disubstituted formamide or the hydrochlorides thereof, of the formula IV 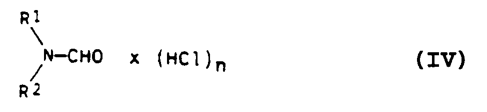 where R¹ and R² are each, independently of one another, C₁-C₃-alkyl or together are a C₄-C₅-alkylene chain which may be interrupted by oxygen or by nitrogen which carries C₁-C₃-alkyl or CHO, and n is 0, 1 or 2, wherein the reaction is carried out with a stationary phase of the catalytic adduct, and the formamide (IV) is loaded with from 0.1 to 95 mol % of phosgene.

2. A process as claimed in claim 1, wherein the reaction is carried out stepwise or continuously, and the formamide (IV) is loaded with from 0.1 to 95 mol % of phosgene.

3. A process as claimed in claim 1, wherein the reaction is carried out stepwise or continuously.

4. A process as claimed in claim 1, wherein the N,N-dialkylformamide is N,Ndimethylformamide, N-methyl-N-ethylformamide or N,N-diethylformamide.

5. A process as claimed in claim 1, wherein the N,N-dialkylformamide is N,N-diethylformamide.

## Revendications

1. Procédé de préparation de chlorures d'acides carboxyliques de la formule générale I : 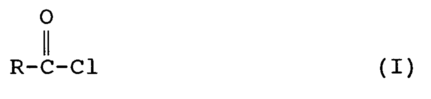 dans laquelle R représente un radical alkyle en C₁ à C₃₀, alcényle en C₂ à C₃₀ et alcynyle en C₂ à C₃₀, à partir de proportions sensiblement équimolaires d'un acide carboxylique de la formule générale II : 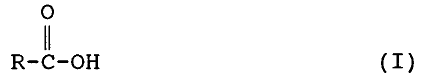 dans laquelle R possède les significations qui lui ont été attribuées ci-dessus et de phosgène COCl₂ (III), en présence d'un adduit d'un catalyseur formé de phosgène et de formamide N,N-disubstitué, ou de ses chlorhydrates de la formule générale IV : 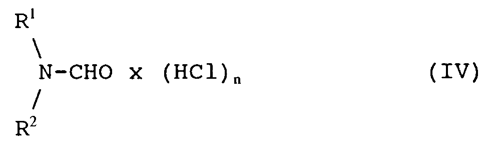 dans laquelle R¹ et R² représentent, chacun indépendamment l'un de l'autre, un radical alkyle en C₁ à C₃, ou bien R¹ et R² forment ensemble une chaîne alkylène en C₄ ou en C₅, ou peut éventuellement être interrompue par un atome d'oxygène ou un atome d'azote, qui porte un groupe CHO ou alkyle en C₁ à C₃ et n est égal à 0, 1 ou 2, caractérisé en ce que l'on entreprend la réaction avec une phase immobile de l'adduit du catalyseur et on charge le formamide (IV) de phosgène jusqu'à raison de 0,1 à 95% molaires.

2. Procédé suivant la revendication 1, caractérisé en ce que l'on entreprend la réaction par étapes ou de manière continue et on charge le formamide (IV) de phosgène jusqu'à raison de 0,1 à 95% molaires.

3. Procédé suivant la revendication 1, caractérisé en ce que l'on entreprend la réaction par étapes ou de manière continue.

4. Procédé suivant la revendication 1, caractérisé en ce que l'on utilise, à titre de N,N-dialkylformamide, le N,N-diméthylformamide, le méthyléthylformamide ou le diéthylformamide.

5. Procédé suivant la revendication 1, caractérisé en ce que l'on utilise le N,N-diéthylformamide à titre de N,N-dialkylformamide.
